# DEMANDE DE BREVET EUROPEEN

(11) **EP 3 482 704 A1**
(43) Date de publication de la demande: **15.05.2019**
(21) Numéro de dépôt: 18205334.8
(22) Date de dépôt: 09.11.2018
(51) Int. Cl.: A61B 17/84, A61B 17/70

(54) **ELEMENT LONGILIGNE TEXTILE SOUPLE PLAT COMPRENANT UN DISPOSITIF D'IDENTIFICATION DE SES FACES A ET B OPPOSEES**

(30) Priorité: 10.11.2017 FR 1760600
(71) Demandeur: Cousin Biotech, 59117 Wervicq Sud (FR)
(72) Inventeur: NOEL, Stéphane, 59496 HANTAY (FR)
(74) Mandataire: Cabinet Beau de Loménie

(57) **Abrégé**

Dispositif implantable (1) comprenant un élément longiligne textile souple (3) plat ayant une première face A (15) et une seconde face B (16) et des première et seconde extrémités libres (11,13) ainsi qu'un dispositif d'identification (20) configuré pour identifier, lorsque ledit élément longiligne (3) forme au moins une portion de boucle (5) délimitant un volume interne Vi, une première position dans laquelle la seconde face interne B (16) est orientée en regard du volume interne Vi dans ladite portion de boucle (5), la seconde face interne B (16) de la première extrémité libre (11) étant en regard directement de la seconde face interne B (16) de la seconde extrémité libre (13) ; une seconde position dans laquelle la première face externe A (15) est orientée en regard du volume interne Vi selon au moins une partie de la portion de boucle (5), la seconde face interne B (16) de la première extrémité libre (11) étant en regard directement de la première face externe A (15) de la seconde extrémité libre (13).

## Description

La présente invention concerne un dispositif implantable comprenant un élément longiligne textile souple plat ainsi qu'un dispositif d'identification permettant de déterminer si l'élément longiligne, lorsqu'il est passé autour d'une portion osseuse, ou d'un autre corps, est vrillé.

Les éléments longilignes textiles plats peuvent être implantés seuls, ou en combinaison avec un autre dispositif implantable avec lequel ils coopèrent, par exemple afin d'assurer la liaison entre un corps osseux et une tige de distraction. De tels éléments longilignes textiles offrent de bonnes résistances mécaniques du fait de leur construction mettant en oeuvre plusieurs fils.

L'élément longiligne plat implanté peut former une ou plusieurs boucles. Or, il est important que ledit élément longiligne ne soit pas vrillé sur sa trajectoire d'implantation. En effet, la zone vrillée peut former une zone ayant un effet abrasif sur les tissus environnants. De plus, les performances mécaniques offertes par l'élément longiligne ne sont pas optimisées dans une mauvaise position d'implantation. Il n'est souvent pas possible pour le praticien de vérifier la position adoptée par l'élément longiligne plat car ce dernier est passé dans des tissus à l'aveugle à l'aide d'outils chirurgicaux. Une fois l'élément longiligne plat implanté, seules ses extrémités sont visibles et préhensibles afin qu'elles soient solidarisées ensemble et/ou à un dispositif permettant leur solidarisation. L'élément longiligne plat est également généralement mis en tension, laquelle tension lorsqu'elle est exercée sur une portion vrillée peut amplifier l'effet de cisaillement sur les tissus. Par ailleurs, l'un des avantages d'un élément longiligne textile plat comparativement à un élément longiligne circulaire, est que ses faces planes ne génèrent pas naturellement d'effet de cisaillement.

EP 0 554 653 A2 a pour objet une tresse implantable circulaire comprenant des fils de couleur. Ces fils de couleur ont pour fonction de permettre de distinguer une tresse d'une autre, les fils de couleur étant associés à des performances déterminées pour chacune des tresses. De plus, EP 0 554 653 A2 ne cherche pas à déterminer la position vrillée d'une tresse, laquelle n'est pas possible du fait de la forme circulaire de la tresse.

### Objet et résumé de l'invention

La présente invention vise à proposer un dispositif implantable comprenant un élément longiligne textile souple plat permettant de déterminer, lorsqu'il forme une portion de boucle, s'il est vrillé afin d'éviter tout risque de cisaillement des tissus et/ou des os autour desquels il est disposé.

La présente invention vise à proposer un dispositif implantable simple d'utilisation, et simple à fabriquer, sans diminuer sa résistance mécanique, notamment à rupture.

La présente invention, pallie les problèmes précités, en ce qu'elle a pour objet, un dispositif implantable comprenant un élément longiligne textile souple plat ayant une première face A et une seconde face B opposée à la première face A, ainsi que des première et seconde extrémités libres. Avantageusement, le dispositif implantable comprend un dispositif d'identification configuré pour identifier, lorsque ledit élément longiligne forme au moins une portion de boucle, en particulier en passant autour au moins d'une partie osseuse, ladite portion de boucle délimitant un volume interne Vi,
- une première position de la portion de boucle dans laquelle la seconde face interne B est orientée en regard du volume interne Vi dans ladite portion de boucle, la seconde face interne B de la première extrémité libre étant en regard directement de la seconde face interne B de la seconde extrémité libre ;
- une seconde position de la portion de boucle dans laquelle la première face externe A est orientée en regard du volume interne Vi selon au moins une partie de la portion de boucle, la seconde face interne B de la première extrémité libre étant en regard directement de la première face externe A de la seconde extrémité libre.

Avantageusement, si l'élément longiligne est vrillé dans ladite portion de boucle, il est possible par l'intermédiaire du dispositif d'identification en superposant les deux extrémités libres, dans le prolongement sensiblement rectiligne de la portion de boucle, de vérifier l'orientation de la première face externe A par rapport à la seconde face interne B, et ce, sans vérifier la disposition de la portion de boucle directement. Grâce au dispositif d'identification, l'analyse du sens des faces opposées planes A et B de l'élément longiligne permet d'identifier si l'élément longiligne est vrillé dans la portion de boucle.

Il n'est pas possible en effet dans l'état de la technique de différencier les deux faces opposées planes d'un élément longiligne textile implantable.

Dans la présente invention, si l'élément longiligne formant au moins une portion de boucle n'est pas vrillé, la première face externe A conserve sensiblement la même orientation selon le trajet dudit élément longiligne, au moins dans ladite portion. La première face externe A reste ainsi orientée vers l'extérieur du volume interne Vi formé par ladite au moins une portion de boucle. En parallèle, la seconde face interne B conserve également la même orientation selon le trajet de l'élément longiligne, au moins dans ladite portion. La seconde face interne B reste ainsi orientée vers l'intérieur du volume interne Vi.

Par contre, si l'élément longiligne formant au moins une portion de boucle est vrillé dans ladite portion, la première face externe A, et donc également la seconde face interne B, sont inversées dans ladite portion. La première face externe A se retrouve orientée vers l'intérieur de ladite au moins une portion de boucle, en particulier orientée en regard du volume interne Vi. La seconde face interne B est, quant à elle orientée vers l'extérieur du volume interne Vi de la portion de boucle. Le dispositif d'identification selon l'invention permet d'identifier si les secondes faces internes B ne sont pas orientées directement en regard l'une de l'autre, au niveau des extrémités libres de l'élément longiligne, en sortie de la portion de boucle, et donc d'en déduire que l'élément longiligne est vrillé dans la portion de boucle.

L'élément longiligne textile est souple car il peut être enroulé, et donc envelopper parfaitement une portion d'un corps osseux ou un muscle ou encore un autre organe.

L'élément longiligne présente par définition une longueur nettement plus grande que sa largeur, par exemple une longueur de l'ordre de 10 cm à 50 cm.

De préférence, l'élément longiligne a une largeur I supérieure à 0 mm et inférieure ou égale à 30 mm, encore de préférence inférieure ou égale à 20 mm, notamment supérieure ou égale à 5mm.

De préférence, l'élément longiligne a une épaisseur supérieure à 0 mm et inférieure ou égale à 10 mm, encore de préférence inférieure ou égale 8 mm, préférentiellement inférieure ou égale à 5 mm.

L'élément longiligne textile est obtenu par la mise en oeuvre de fils et/ou d'élément(s) longiligne(s) auxiliaire(s) (par exemple une ou plusieurs tresse(s), ruban(s),...) sur un métier textile (notamment un métier à tricoter, à tisser (de faible laize), ou à tresser).

L'élément longiligne textile peut comprendre un revêtement polymère, bien que de préférence il n'en comprenne pas.

Le dispositif d'identification est configuré pour qu'un utilisateur puisse différencier la première face A par opposition à la seconde face B. Ce dispositif d'identification peut ainsi comprendre un moyen d'identification visuel et/ou un moyen d'identification tactile. Par exemple, l'une des deux faces A et B peut comprendre des éléments en reliefs alors que l'autre face est sensiblement lisse au toucher.

Par exemple, l'une des deux faces A et B peut comprendre un premier motif coloré alors que l'autre face comprend un second motif, différent du premier motif, ou n'en comprend pas.

L'élément longiligne textile comprend un ou des fil(s) multifilamentaires et/ou un ou des fil(s) monofilamentaires et/ou un ou des fil(s) filés de fibres, de préférence choisi(s) dans un ou des matériau(x) polymère(s) synthétique(s), résorbable(s) ou non, encore de préférence le ou lesdits matériau(x) polymère(s) est/sont choisi(s) parmi : les polyesters, tel que le polyéthylène téréphtalate; les polyamides, tels que le PA 6, le PA 6-6, le PA 4-6, le PA 11 ou 12 ; les polyoléfines, tels que le polypropylène, et le polyéthylène ; de préférence le polypropylène ; les polymères d'acide lactique, et notamment l'acide polylactique de forme L ou D ou leur mélange (PLLA, PLA, PDLA) ; ou leur mélange.

De préférence, le ou les fil(s) monofilamentaire(s) ont un diamètre externe supérieur à 0 mm, et inférieur ou égal à 5 mm, encore de préférence inférieur ou égal à 2 mm, préférentiellement inférieur ou égal à 1 mm, notamment inférieur ou égal à 0,5 mm.

De préférence, le ou les fil(s) multifilamentaire(s) a/ont un titre supérieur à 50 dtex, notamment inférieur ou égal à 2000 dtex.

L'élément longiligne peut être creux, dans ce cas il comprend deux couches textiles superposées, ou ne pas être creux et être formé dans ce cas d'une seule couche textile.

L'élément longiligne textile peut être plat et creux en sortie du métier textile, par exemple en sortie d'un métier à tisser de faible laize, ou en sortie d'un métier à tresser, ou encore être sous forme de tube en sortie d'un métier à tresser ou à tricoter. Dans ce dernier cas, l'élément longiligne textile subit un traitement thermique afin de l'aplatir et lui conférer des faces A et B planes et opposées.

Lorsque l'élément textile comprend deux couches textiles superposées, les première et seconde faces A et B correspondent aux faces externes des deux couches textiles. Les surfaces internes des deux couches textiles sont orientées en regard l'une de l'autre et débouchent dans le volume interne dudit élément longiligne creux.

Dans une variante, le dispositif d'identification est de construction textile unitaire avec l'élément longiligne textile, en particulier de construction tricotée, tressée ou tissée, unitaire avec l'élément longiligne textile tricoté, tressé ou tissé respectivement.

Avantageusement, le dispositif d'identification est disposé dans l'élément longiligne lors de sa fabrication sur un métier textile. Cette disposition facilite la fabrication de l'élément longiligne et assure une bonne reproductibilité dans la disposition du dispositif d'identification sur la longueur de l'élément longiligne.

Le dispositif d'identification contribue également à la résistance mécanique de l'élément longiligne textile.

Le dispositif d'identification est de préférence choisi parmi : un ou plusieurs fil(s) multifilamentaire(s) et/ou monofilamentaire(s) et/ou fil(s) filés de fibres, un ou plusieurs ruban(s), une ou plusieurs tresse(s), le ou lesquels est/sont éventuellement coloré(s), en particulier dans une ou des couleur(s) différente(s) de la ou des couleur(s) mise(s) en oeuvre dans le reste de l'élément longiligne.

La disposition par exemple d'un fil coloré tressé avec un ensemble d'autres fils non colorés également tressés dans une tresse n'est pas suffisante pour former un dispositif d'identification selon l'invention. En effet, l'agencement du dispositif d'identification, et donc du fil coloré, doit être déterminé de telle manière que la première face externe A puisse être différenciée de la seconde face interne B.

Dans une variante de réalisation, le dispositif d'identification comprend des moyens d'identification visuels et/ou tactiles de la première face externe A et de la seconde face interne B, lesdits moyens étant tressés, tricotés ou tissés lors du tricotage, du tressage ou du tissage de l'élément longiligne textile.

Dans une variante, le dispositif d'identification est disposé sur la première face externe A et/ou sur la seconde face interne B, de façon continue ou discontinue, sur toute la longueur de l'élément longiligne textile.

Dans une variante, le dispositif d'identification est disposé selon l'un des deux bords longitudinaux de l'élément longiligne, à la fois sur la première face externe A et sur la seconde face interne B.

Le dispositif d'identification est disposé selon le premier bord ou la second bord longitudinal de la première face externe A, et en sorte de retrouver en vis-à-vis également sur la seconde face interne B, selon le premier bord longitudinal ou le second bord longitudinal respectivement.

Dans ce cas, le dispositif d'identification est disposé selon la moitié longitudinale droite ou la moitié longitudinale gauche de l'une des faces A et B et n'est pas centré sur la largeur de l'élément longiligne. Il ne serait pas en effet possible de distinguer les faces A et B dans ce dernier cas.

Dans une variante, le dispositif d'identification comprend un ou plusieurs élément(s) longiligne(s) auxiliaire(s) souple(s), tel qu'un ou plusieurs fil(s) monofilamentaire(s) et/ou multifilamentaire(s) et/ou filés de fibres, une ou plusieurs tresse(s), un ou plusieurs ruban(s), ou leur mélange.

Dans une variante, l'élément longiligne textile plat est une tresse creuse.

Dans une variante, l'élément longiligne textile est une tresse triaxiale comprenant un premier ensemble d'au moins un fil, tressé selon un axe L1, un second ensemble d'au moins un fil, tressé selon un axe L2, et le dispositif d'identification, tressé selon un axe L3, lesdits axes L1, L2 et L3 étant différents.

Le dispositif d'identification est tressé avec les premier et second ensembles de fils.

De préférence, l'élément longiligne comprend, en particulier est constitué, des premier et second ensembles de fils, ainsi que d'un troisième ensemble d'au moins un fil, tressé selon l'axe L3. Encore de préférence le troisième ensemble d'au moins un fil est disposé sur la première face externe A, et éventuellement sur la seconde face interne B et le dispositif d'identification est disposé sur la seconde face interne B, en particulier le dispositif d'identification n'est pas disposé sur la première face externe A.

Le dispositif d'identification peut être formé par un ou plusieurs fil(s), tels que définis dans le présent texte, éventuellement colorés dans une ou plusieurs couleur(s) différente(s) de la ou des couleur(s) des fils des premier, second, et éventuellement troisième, ensembles de fils.

Avantageusement, il est possible de disposer de manière privilégiée un ou plusieurs fil(s) déterminé(s) selon uniquement une des deux faces A et B.

L'élément longiligne a un axe longitudinal central A1 et un axe transversal T, sensiblement perpendiculaire à l'axe A1.

L'axe longitudinal central A1 passe au milieu des faces A et B.

Les axes L1 et L2 forment respectivement des angles +α1 et -α2 avec l'axe A1 (avec |α1| ≠ |α2| ou |α1| = |α2|), lesquels angles |α1| et |α2| sont supérieur à 0° et inférieur à 180°, notamment inférieur à 90°, en particulier inférieur à 50°, notamment de l'ordre de 45° à +/- 5°.

De préférence, l'axe A1 est sensiblement parallèle à l'axe L3.

Dans une variante de réalisation, l'élément longiligne textile comprend des première et seconde couches textile superposées, la face externe de la première couche textile est formée de la première face externe A, et la face externe de la seconde couche textile est formée de la seconde face interne B.

Les faces internes de la première couche textile et de la seconde couche textile sont en regard l'une de l'autre, et débouchent dans le volume interne délimité entre lesdites première et seconde couches.

Les première et seconde couches textile superposées sont de construction textile unitaire, en particulier obtenues par le tressage d'une tresse creuse.

Dans un mode de réalisation, la première couche textile comprend le dispositif d'identification, notamment débouchant sur la face externe de ladite première couche, et éventuellement sur la face interne de cette dernière.

Lorsque l'élément longiligne textile est une tresse creuse triaxiale, le dispositif d'identification est disposé selon l'axe L3 sur la première couche tressée, en particulier sans se retrouver sur la seconde couche tressée.

Dans un mode de réalisation, la première couche tressée est obtenue par le tressage des premier et second ensembles de fils avec le dispositif d'identification, et éventuellement le troisième ensemble de fils. De préférence, la seconde couche tressée est obtenue par le tressage des premier, second et troisième ensembles de fils.

Dans une variante, l'élément longiligne textile plat a un axe longitudinal A1, et le dispositif d'identification à une trajectoire sensiblement rectiligne selon l'axe L3, l'axe L3 et l'axe longitudinal A1 étant parallèles.

Dans une variante, le dispositif d'identification comprend des boucles proéminentes, disposées de manière continue ou discontinue, sur la première face externe A et/ou la seconde face externe B, de préférence lesdites boucles sont constituées d'un fil ou de plusieurs fils multifilamentaire(s).

De préférence, la tension appliquée lors du la mise en oeuvre textile, notamment par tressage, et/ou l'allongement à rupture du ou des fil(s) multifilamentaire(s) formant le dispositif d'identification est/sont différent(e)(s), notamment supérieur(e)(s), à la tension appliquée et/ou à l'allongement à rupture du ou des autres fil(s) formant l'élément longiligne textile permettant ainsi de créer des excès de longueurs de fils formant des boucles se projetant de la face A et/ou de la face B, de préférence selon une seule des deux faces A et B.

### Brève description des dessins

L'invention et ses avantages seront mieux compris à la lecture de la description détaillée des exemples de réalisation particuliers, pris à titre d'exemples nullement limitatif et illustrés par les dessins annexés sur lesquels :
- la figure **1** est une représentation schématique d'un premier exemple de dispositif implantable selon l'invention disposé sur une moins une portion osseuse dans la première position ;
- la figure **2A** est une représentation schématique du premier exemple de dispositif implantable seul dans la première position ;
- la figure **2B** est une représentation schématique du premier exemple de dispositif implantable seul dans la seconde position ;
- la figure **2C** est une représentation schématique du mode de tressage de la première couche de l'élément longiligne textile plat du premier exemple de dispositif implantable ;
- la figure **3** est une représentation schématique selon le plan de coupe III-III représenté à la figure **2A** de l'élément longiligne textile plat du premier exemple de dispositif implantable ;
- la figure **4A** est une représentation schématique d'un second exemple de dispositif implantable seul dans la première position ;
- la figure **4B** est une représentation schématique du second exemple de dispositif implantable seul dans la seconde position ;
- la figure **5A** est une représentation schématique d'un troisième exemple de dispositif implantable seul dans la première position ;
- la figure **5B** est une représentation schématique du troisième exemple de dispositif implantable seul dans la seconde position.

### Description détaillée de l'invention

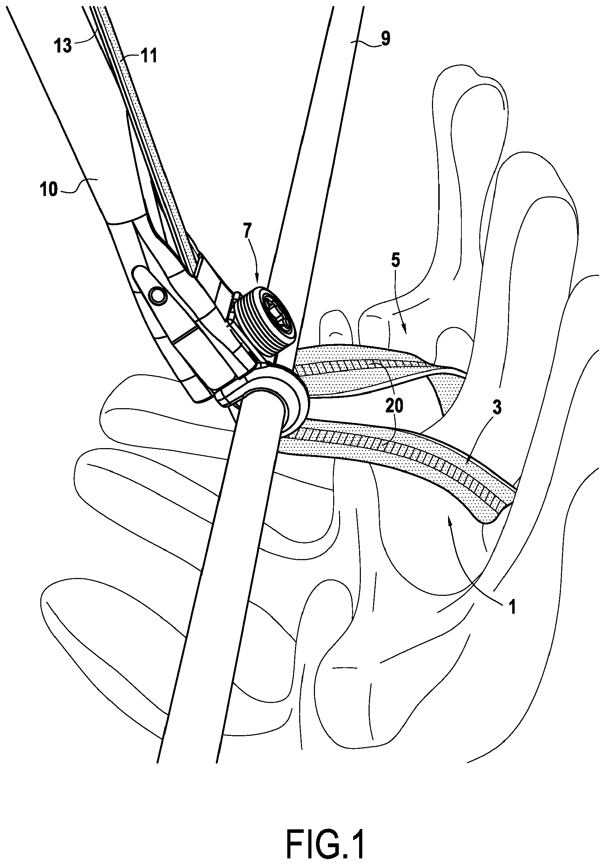

La figure **1** illustre la disposition d'un premier exemple de dispositif implantable **1** selon l'invention dans lequel l'élément longiligne textile plat et souple **3** est disposé autour d'au moins une portion d'un corps vertébral en formant une portion de boucle **5** dans la première position. L'élément longiligne textile **3,** dans cet exemple précis, coopère avec un connecteur **7** et une tige implantable **9,** il peut néanmoins être utilisé seul ou en combinaison avec d'autres implants. Par ailleurs, l'élément longiligne **3** peut former une boucle **5** autour d'un autre type de corps, osseux ou non, par exemple autour d'un muscle ou d'un organe. Les extrémités libres **11** et **13** de l'élément longiligne **3,** après que ce dernier ait été mis en tension grâce au dispositif de mis en tension **10** représenté partiellement, sont solidarisées à l'aide du connecteur **7.**

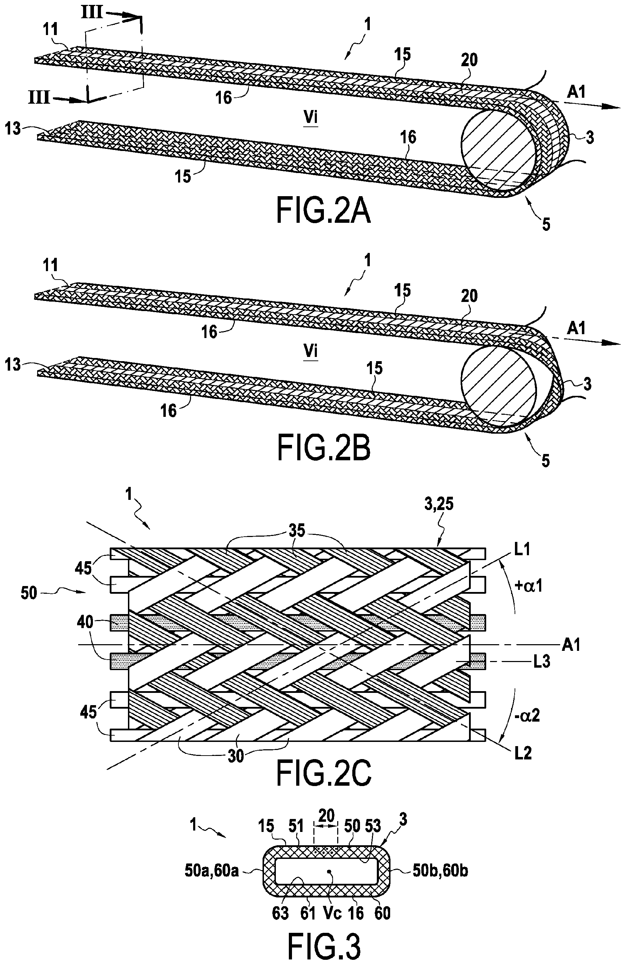

L'élément longiligne textile souple plat **3,** représenté aux figures **1** et **2A** à **2C,** a une première face A **15** et une seconde face B **16** opposée à la première face A **15,** et des première et seconde extrémités libres **11,13.** L'élément longiligne textile **3** a un axe longitudinal central **A1,** et un axe transversal T.

Le dispositif implantable **1** comprend un dispositif d'identification **20** configuré pour identifier, lorsque ledit élément longiligne **3** forme au moins une boucle **5,** en particulier en passant autour au moins d'une portion osseuse, ladite boucle **5** délimitant un volume interne Vi,
- une première position de la boucle **5** dans laquelle la seconde face interne B **16** est orientée en regard du volume interne Vi dans ladite boucle **5,** la seconde face interne B **16** de la première extrémité libre **11** étant en regard directement de la seconde face interne B **16** de la seconde extrémité libre **13** ; et
- une seconde position de la boucle **5** dans laquelle la première face externe A **15** est orientée en regard du volume interne Vi selon au moins une portion de la boucle **5,** la seconde face interne B **16** de la première extrémité libre **11** étant en regard directement de la première face externe A **15** de la seconde extrémité libre **13.**

Le dispositif d'identification **20** est de construction textile unitaire avec l'élément longiligne textile **3,** en particulier de construction tressée unitaire avec l'élément longiligne textile **3** tressé.

Le dispositif d'identification **20** comprend au moins un moyen d'identification visuel **22** (voir figure **2C**) et/ou tactile, dans cet exemple précis visuel, de la première face externe A **15,** ledit au moins un moyen d'identification **22** étant tressé lors du tressage de l'élément longiligne textile **3.**

Le dispositif d'identification **20** est disposé sur la première face externe A **15,** de façon continue ou discontinue, dans cet exemple précis de façon continue, sur toute la longueur de l'élément longiligne textile **3.**

L'élément longiligne textile **3** plat est une tresse creuse triaxiale **25** obtenue par le tressage d'un premier ensemble **30** de fils selon un axe **L1,** d'un second ensemble **35** de fils selon un axe **L2,** d'un troisième ensemble **45** de fils selon l'axe **L3,** et du dispositif d'identification **20** selon l'axe **L3,** lesdits axes **L1, L2** et **L3** étant différents. Le mode d'assemblage des différents ensembles de fils **30,35,45** et dispositif d'identification **20** étant représenté de manière schématique à la figure **2C****.**

Le dispositif d'identification **20** comprend en particulier une pluralité de fils **40,** notamment multifilamentaires, distincts visuellement et/ou tactilement, des fils des premier, second et troisième ensembles **30,35,45.** Dans cet exemple précis, les fils **40** sont dans une couleur, par exemple en bleu, différente de la ou des couleur(s) des fils des premier, second et troisième ensembles **30,35,45,** ces derniers sont notamment blancs.

Le dispositif d'identification **20** à une trajectoire sensiblement rectiligne selon l'axe **L3,** l'axe **L3** et l'axe longitudinal central **A1** étant parallèles.

L'élément longiligne textile **3** comprend deux couches tressées **50,60** reliées selon leurs bords longitudinaux **50a, 60a**; **50b,60b** lors du tressage et délimitant un volume interne creux **Vc** (figure **3**)**.** La première couche tressée **50** comprend une face externe **51** formant la première face externe A **15** et une face interne **53** débouchant dans le volume interne **Vc.** La seconde couche tressée **60** comprend une face externe **61** formant la seconde face interne B **16** et une face interne **63** débouchant également dans le volume interne **Vc.** Les faces internes **53** et **63** des première et seconde couches **50,60** sont en regard l'une de l'autre.

Seule une partie de la première couche tressée **50** de la tresse triaxiale **25** est représentée à la figure **2C****,** ladite couche **50** comprend ainsi le dispositif d'identification **20** qui sera visible à la fois sur sa face externe **51,** et donc la première face externe A **15,** et sur sa face interne **53.** La première couche **50** comprend des fils du troisième ensemble de fils **45** tel que représenté à la figure **2C****.** La première couche **50** peut également ne pas comprendre de fils du troisième ensemble de fils **45,** et dans ce cas, le dispositif d'identification **20** comprend davantage de fils pour remplacer ledit troisième ensemble de fils **45.**

La seconde couche **60** de la tresse triaxiale **25** comprend dans cet exemple précis, les trois ensembles de fils **30,35,45** tressés selon les axes **L1, L2** et **L3** et ne comprend pas de dispositif d'identification **20.**

Dans cet exemple précis α1 est de l'ordre de +30° et α2 est de l'ordre de -30°. Dans une autre variante préférée non illustrée, α1 est de l'ordre de +45° et α2 est de l'ordre de -45°.

Dans un exemple préféré, le premier ensemble **30** comprend 24 fils, notamment de couleur banche, chaque fils étant formé de six fils multifilamentaires de 138 dtex chacun (lesquels sont disposés de manière parallèle ou légèrement retordus), notamment en polyéthylène téréphtalate (PET), soit un total de 19 872 dtex (= 24*(6*138)). Le second ensemble **35** comprend 24 fils, notamment de couleur blanche, chaque fil étant formé de six fils multifilamentaires de 138 dtex chacun (lesquels sont disposés de manière parallèle ou légèrement retordus), soit un total de 19 872 dtex (= 24*(6*138)). Le troisième ensemble **45** comprend douze fils, chaque fil étant formé de six fils multifilamentaires de 138 dtex chacun (lesquels sont disposés de manière parallèle ou légèrement retordus), soit un total de 9 936 dtex (=12*6*138 dtex), en particulier en PET, ces fils sont disposés sur la seconde face interne B **16.** Le dispositif d'identification **20** comprend douze fils, chaque fil étant formé de six fils multifilamentaires de 138 dtex chacun (lesquels sont disposés de manière parallèle ou légèrement retordus), en particulier en PET, soit un total de 9 936 dtex -12*6*138 dtex). Ces fils sont notamment teints dans la masse, et disposés sur la première face externe A **15,** laquelle ne comprend pas dans cet exemple préféré de fils du troisième ensemble de fils **45** sur la première couche **50.** Les fils du troisième ensemble de fils **45** sont ainsi disposés sur la seconde couche **60.**

Le tressage triaxial des trois ensembles de fils **30,35,40** et du dispositif d'identification est avantageusement configuré de sorte que les fils formant le dispositif d'identification soient disposés sur une seule des deux couches tressées permettant ainsi d'identifier visuellement de manière certaine la première face externe A de la seconde face interne B.

En fonctionnement, l'élément longiligne textile plat **3** est disposé autour du corps vertébral représenté à la figure **1****,** puis ses extrémités **11,13** sont réunies et superposées dans le prolongement de la boucle **5,** sans les vriller bien sûr. Si la seconde face interne B **16** de la première extrémité libre **11** est en regard directement de la seconde face interne B **16** de la seconde extrémité libre **13,** le dispositif implantable **1** est dans la première position et n'est donc pas vrillé. Si par contre, la seconde face interne B **16** de la première extrémité libre **11** est en regard directement de la première face externe A **15** de la seconde extrémité libre **13,** le dispositif implantable **1** est dans la seconde position, et est vrillé dans la portion de boucle **5.** Il est alors possible de corriger la position de la portion de boucle **5** puis de contrôler que l'élément longiligne **3** est désormais dans la première position.

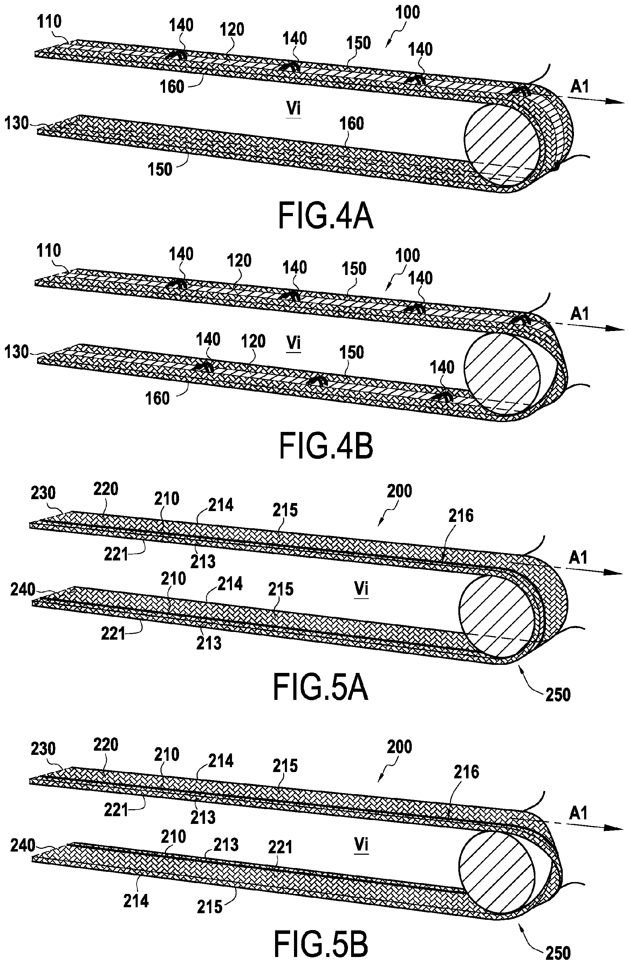

Le second exemple de dispositif implantable **100** représenté aux figures **4A** et **4B** diffère du premier exemple de dispositif implantable **1** par son dispositif d'identification **110.** Le dispositif d'identification **110** est également formé par plusieurs fils tressés **120** avec des premier, second et troisième ensembles de fils pour former une tresse triaxiale **130.** Les fils **120** formant le dispositif d'identification **110** sont cependant tressés avec une tension inférieure à celle appliquée aux fils des premier, second et troisième ensembles de fils de sorte que les fils **120** du dispositif d'identification **110** forment des boucles **140** à intervalles réguliers. Le dispositif d'identification **120** est donc ici visuel mais également tactile. Les boucles proéminentes **140** améliorent également l'identification de la première face externe A **150** comparativement à la seconde face interne B **160.**

Le troisième exemple de dispositif implantable **200** représenté aux figures **5A** et **5B** comprend un dispositif d'identification **210** disposé selon l'un des premier et second bords longitudinaux **213,214** de l'élément longiligne plat **215,** dans cet exemple précis selon le première bord longitudinal **213** sur la première face externe A **220,** et sur la seconde face interne B **221.** L'élément longiligne textile plat **215** est une tresse plate non creuse, comprenant une seule couche. Lorsque l'élément longiligne plat **215** est dans la première position, le dispositif d'identification **210** est disposé selon le même premier bord longitudinal **213** aux niveaux de ses extrémités libres **230,240.** Par contre, si l'élément longiligne **215** est vrillé dans la portion de boucle **250,** le dispositif d'identification **210** se retrouve visuellement comme s'il était disposé selon le second bord longitudinal **214** de l'élément longiligne **215.**

Les différentes variantes de dispositifs implantables **1,100,200** selon l'invention sont faciles à mettre en oeuvre et de construction simplifiée. Etant de préférence de construction textile unitaire avec l'élément longiligne textile plat **3,130,215,** en particulier lors du tressage, la résistance mécanique de l'élément longiligne **3,130,215** est préservée.

Il est ainsi aisé d'identifier en comparant les faces A et B de l'élément longiligne au niveau des deux extrémités libres si ce dernier est vrillé dans la portion de boucle en amont et ainsi de corriger la disposition de l'élément longiligne jusqu'à ce qu'il soit dans la première position.

## Revendications

1. Dispositif implantable (1,100,200) comprenant un élément longiligne textile souple (3,215) plat ayant une première face A (15,150,220) et une seconde face B (16,160,221) opposée à la première face A, ainsi que des première et seconde extrémités libres (11,13 ; 230,240), **caractérisé en ce que** le dispositif implantable (1,100,200) comprend un dispositif d'identification (20,110,210) configuré pour identifier, lorsque ledit élément longiligne (3,215) forme au moins une portion de boucle (5), en particulier en passant autour au moins d'une partie osseuse, ladite portion de boucle (5) délimitant un volume interne Vi,
- une première position de la portion de boucle (5) dans laquelle la seconde face interne B (16,160,221) est orientée en regard du volume interne Vi dans ladite portion de boucle (5), la seconde face interne B (16,160,221) de la première extrémité libre (11) étant en regard directement de la seconde face interne B (16,160,221) de la seconde extrémité libre (13) ;
- une seconde position de la portion de boucle (5) dans laquelle la première face externe A (15,150,220) est orientée en regard du volume interne Vi selon au moins une partie de la portion de boucle (5), la seconde face interne B (16,160,221) de la première extrémité libre (11) étant en regard directement de la première face externe A (15,150,220) de la seconde extrémité libre (13).

2. Dispositif implantable (1,100,200) selon la revendication **1, caractérisé en ce que** le dispositif d'identification (20,110,210) est de construction textile unitaire avec l'élément longiligne textile (3,215), en particulier de construction tricotée, tressée ou tissée, unitaire avec l'élément longiligne textile tricoté, tressé ou tissé respectivement.

3. Dispositif implantable (1,100,200) selon l'une ou l'autre des revendications **1** et **2, caractérisé en ce que** le dispositif d'identification (20,110,210) comprend au moins un moyen d'identification visuel (22) et/ou tactile de la première face externe A et de la seconde face interne B, ledit moyen étant tressé, tricoté ou tissé lors du tricotage, du tressage ou du tissage de l'élément longiligne textile.

4. Dispositif implantable (1,100,200) selon l'une quelconque des revendications **1** à **3, caractérisé en ce que** le dispositif d'identification (20,110,210) est disposé sur la première face externe A et/ou sur la seconde face interne B, de façon continue ou discontinue, sur toute la longueur de l'élément longiligne textile.

5. Dispositif implantable (200) selon la revendication **4, caractérisé en ce que** le dispositif d'identification (210) est disposé selon l'un des deux bords longitudinaux (213,214) de l'élément longiligne (215), à la fois sur la première face externe A (220) et sur la seconde face interne B (221).

6. Dispositif implantable (1,100,200) selon l'une ou l'quelconque des revendications **1** à **5, caractérisé en ce que** le dispositif d'identification (20,110,210) comprend un élément longiligne auxiliaire souple (40,120,216), tel qu'un ou plusieurs fil(s) monofilamentaire(s) ou multifilamentaire(s), une ou plusieurs tresse(s), un ou plusieurs ruban(s).

7. Dispositif implantable (1,100) selon l'une quelconque des revendications **1** à **6, caractérisé en ce que** l'élément longiligne textile plat est une tresse creuse.

8. Dispositif implantable (1,100) selon l'une quelconque des revendications **1** à **7, caractérisé en ce que** l'élément longiligne textile (3) est une tresse triaxiale (25,130) comprenant un premier ensemble (30) d'au moins un fil tressé selon un axe L1, un second ensemble (35) d'au moins un fil tressé selon un axe L2, et **en ce que** le dispositif d'identification (20,110) est tressé selon un axe L3, lesdits axes L1, L2 et L3 étant différents.

9. Dispositif implantable (1,100) selon l'une quelconque des revendications **1** à **8, caractérisé en ce que** l'élément longiligne textile plat (25,130) a un axe longitudinal A1, et **en ce que** le dispositif d'identification (20,110) à une trajectoire sensiblement rectiligne selon l'axe L3, l'axe L3 et l'axe longitudinal A1 étant parallèles.

10. Dispositif implantable (1,100) selon l'une quelconque des revendications **1** à **9, caractérisé en ce que** l'élément longiligne textile (3) comprend des première et seconde couches textiles (50,60) superposées, la face externe (51) de la première couche textile (50) est formée de la première face externe A (15,150), et la face externe (61) de la seconde couche textile (60) est formée de la seconde face interne B (16,160).

11. Dispositif implantable (100) selon l'une quelconque des revendications **1** à **10, caractérisé en ce que** le dispositif d'identification (20,110) comprend des boucles proéminentes (140), disposées de manière continue ou discontinue, sur la première face externe A (150) et/ou la seconde face externe B (160), de préférence lesdites boucles (140) sont constituées d'un fil ou plusieurs fils multifilamentaire(s) (120).
